# EUROPEAN PATENT APPLICATION

(11) **EP 2 075 246 A1**
(43) Date of publication of application: **01.07.2009**
(21) Application number: 07150451.8
(22) Date of filing: 27.12.2007
(51) Int. Cl.: C07D 207/36, A61K 31/402

(54) **A process for preparation of amorphous form of atorvastatin hemi-calcium salt**

(71) Applicant: M. J. Institute of Research, 400 021 Mumbai (IN)
(72) Inventor: Vasantray, Vyas Ashok, 390 021 Gujarat (IN); Pranlal, Doshi Vinay, 390 023 Gujarat (IN)
(74) Representative: Peebles, Katrina

(57) **Abstract**

The present invention relates to a process for preparation of atorvastatin hemi-calcium salt in its amorphous form.

## Description

The present invention relates to a process for preparation of a pharmaceutical substance.

The active pharmaceutical ingredient known as Atorvastatin hemi-calcium salt, which is chemically known as [R-(R*,R*)]-2-(4-fluorophenyl)-β-δ-dihydroxy-5-[1-methyl-ethyl]-3-phenyl-4-[(phenylamino) carbonyl-1H-pyrrole-1-heptanoic acid hemi-calcium salt is a member of the class of drugs called statins.

The conversion of 3-hydroxy-3-methylglutaryl-coenzyme A (HMG-CoA) to mevalonate is an early and rate-limiting step in the cholesterol biosynthetic pathway. This step is catalyzed by the enzyme HMG-CoA reductase. Statins inhibit HMG-CoA reductase from catalyzing this conversion. As such, statins are considered as potent lipid lowering agents.

Atorvastatin in its hemi-calcium salt form is an excellent inhibitor of the enzyme HMG-CoA reductase, thus it is useful as a hypolipidemic and hypocholesterolemic active pharmaceutical ingredient for the therapy of lipidic metabolic disorders. It is used successfully for preventing and for treating coronary heart disease.

The hemi-calcium salt of atorvastatin has been prepared in the past using several synthetic methods, and it is reported to occur in various polymorphic forms which can be crystalline or amorphous in its physical characteristics.

The preparation of Atorvastatin and its key intermediates which are required in the synthesis are described in various patents such as US Pat nos. 5,003,080; 5,092,045; 5,103,024; 5,124,482; 5,149,837; 5,155,251; 5,216,174; 5,245,047; 5,245,793; 5,280,126; 5,342,952; 5,397,792.

The following account provides a brief review regarding preparation of different forms of atorvastatin hemi-calcium salt, in particular, the amorphous form:
WO 02/43732 describes preparation of several new forms of Atorvastatin hemi-calcium salt as well as new processes in preparation of the same. These forms mainly include various crystalline forms and solvates and hydrates thereof. A process for preparation of amorphous atorvastatin has also been described.
WO 03/068739 describes a method for preparation of atorvastatin hemi-calcium amorphous by interacting a solution containing alkali or ammonium type salt of atorvastatin, with a solution containing calcium ions as a calcium salt, calcium hydroxide etc. The amorphous form is obtained without isolation of any intermediate crystalline or other such forms.
The patent WO 2004/085391 relates to a new process for synthesis of the amorphous variety wherein a salt of atorvastatin acid is first formed with a basic amino acid like L-lysine or L-arginine. The solution of such a salt in a mixture of aqueous organic solvent is then treated with a solution of calcium salt like calcium acetate or calcium chloride. Atorvastatin hemi-calcium salt is isolated by filtration as an amorphous product.
WO 2005/090301 details a new crystalline form R of atorvastatin hemi-calcium salt as well as its hydrates this crystalline form R was also converted to its amorphous form. Further, pharmaceutical compositions with this new crystalline form R along with pharmaceutically acceptable excipients are also mentioned.
WO 02/083637 relates to another process for preparing amorphous atorvastatin calcium in the presence of an aqueous methanollic solvent system. The process involves acidic and alkaline hydrolysis of a diol protected tert butyl ester. The crude amorphous product is isolated first which is then purified by addition of its methanollic solution in an aqueous medium.
US patent 6,613,916 deals with conversion of crystalline atorvastatin calcium into the amorphous form. The crystalline product is first dissolved in relatively polar solvents like methanol, alcohol, acetone and the like, partially concentrated and then added into another relatively less polar solvent like ether so as to obtain the amorphous product which is isolated by filtration. US patent 2003/0149279 also describes a similar process wherein the crystalline product is first dissolved in a non-hydroxyllic solvent like tetrahydrofuran followed by treatment with a relatively non polar anti-solvent like a hydrocarbon.
US patent application 2004/0242670 relates to a process for the preparation of the amorphous form by dissolving the crystalline product in a hydroxyllic solvent followed by complete removal of the solvent by spray drying. The resultant product was found to be of amorphous character. It is also been shown that a crystalline product can be converted into its amorphous form by extended pulverization or grinding. Similar spray drying process to obtain amorphous atorvastatin hemi-calcium salt is also reported in US patent 2005/0032880..
Another process for the preparation of amorphous atorvastatin calcium, as described in US patent 2005/0165242, deals with converting the intermediate atorvastatin lactone into its sodium salt by alkaline hydrolysis. This solution of sodium salt is next treated with aqueous solution of a calcium salt like calcium chloride or calcium acetate, which may be added up with seeds of the amorphous material. Resultant solid is isolated by filtration and drying to obtain amorphous product.
WO 03/099785 indicates the use of aliphatic acyclic ketone type solvent like acetone, butanone, pentanone, hexanone etc., followed by dissolution at elevated temperature, filtration and finally desolventisation. Similarly, US patents 6,087,511 and 6,274,740 describe the preparation of atorvastatin calcium amorphous from the crystalline form I by dissolution in a non-hydroxyllic solvent followed by filtration and complete de-solventization in vacuum. US patent 2006/0106230 also describes preparation of the amorphous product by dissolution in certain organic solvents comprised of mixture of hydroxyllic solvent with other ketonic or ester type solvents and then removal of the solvent by techniques like spray drying, rapid vacuum evaporation and thin film evaporation.
According to WO 2006/048888 the amorphous substance can be prepared by using solvents like 1,4-dioxane; acetonitrile; toluene; anisole; tert-butanol etc. in combination with anti-solvents like hydrocarbons and ethers. The amorphous product is isolated by usual vacuum filtration and vacuum drying.
A process according to WO 2006/021969 employs alkaline hydrolysis of the diol protected tert butyl ester i.e. compound of formula (I) in tetrahydrofuran-water mixture. Resultant sodium salt of atorvastatin is isolated by filtration followed by re-dissolution in an organic ester solvent like ethyl acetate. The ethyl acetate solution is next treated with aqueous solution of calcium salt, separated and then desolventized to obtain the amorphous product.
According to US patent 6,646,133 amorphous atorvastatin calcium is obtained from the crude substance by dissolving it in alcoholic solvent followed by filtration and cooling. The precipitated amorphous substance is isolated by filtration. The patent suggests that it may not be essential to have totally non-hydroxyllic solvent for obtaining the amorphous product.
US patent 6,867,306 describes the use of the intermediate viz. phenyl boronate diol protecting pyrrole derivative for synthesis of atorvastatin calcium. This is hydrolysed to obtain atorvastatin sodium in aqueous solution, and then converted into atorvastatin acid followed by its extraction in an organic solvent. The acid is next derivatised to its ammonium salt, purified by crystallization. It is re-dissolved in an organic solvent mixture, and finally treated with aqueous calcium salt solution, whereby, amorphous atorvastatin calcium precipitates out.
US patent application 2006/0128971 describes the preparation of amorphous atorvastatin hemi-calcium salt by use of solvents like tetrahydrofuran, dimethyl acetamide, dimethyl sulfoxide etc. to dissolve crude amorphous atorvastatin calcium and then adding the solution in demineralised water. The solid product is filtered and vacuum dried to obtain purified amorphous product.
US patent application 2006/0142592 describes the preparation of certain crystalline as well as amorphous forms of atorvastatin. Further, preparation of an impurity substance atorvastatin calcium epoxy dihydroxy is also mentioned. Novel salt forms of atorvastatin acid and their method of preparation are described in WO 2005/105738.
Atorvastatin salts are prepared while using basic or amine compounds like ammonia, benethamine, benzathime, dibenzyl amine, diethyl amine, erbumine, L-lisyne, morpholine, piperazine. These salts have also been shown to be useful as agents for treatment of hyperlipidemia, hypercholesterolemia, osteoporosis, benign prostatic hyperplasia and Alzheimer's Disease.

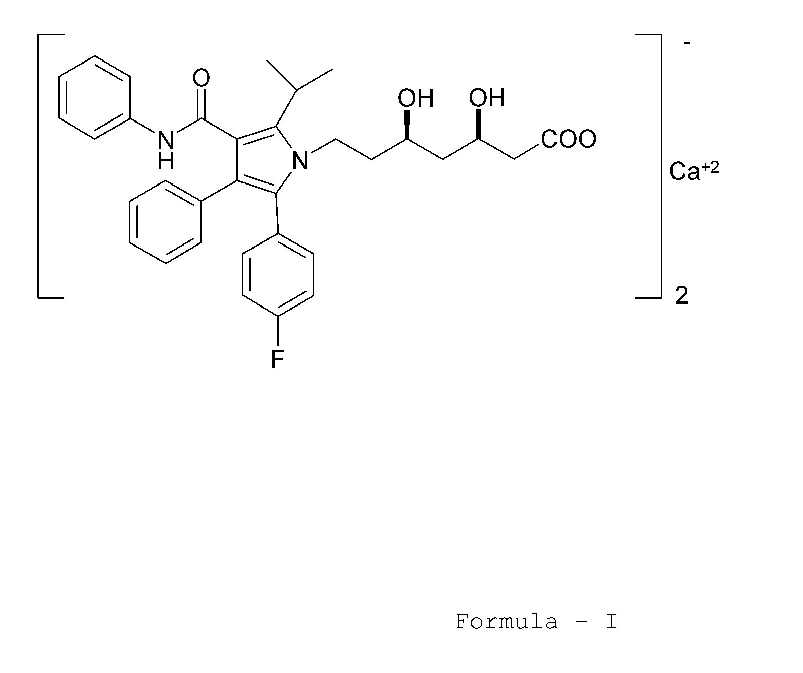

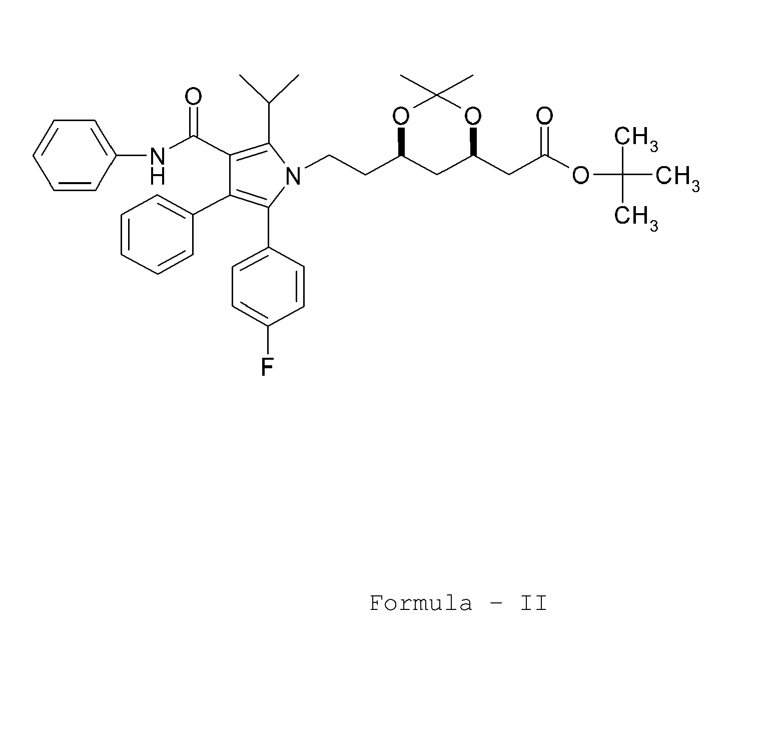

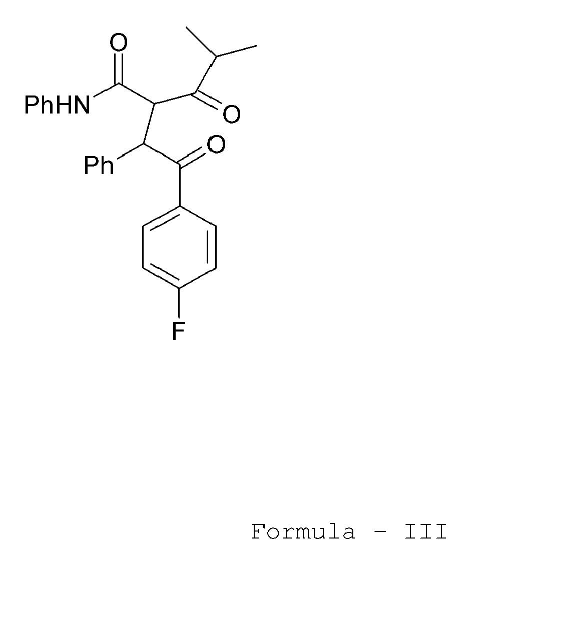

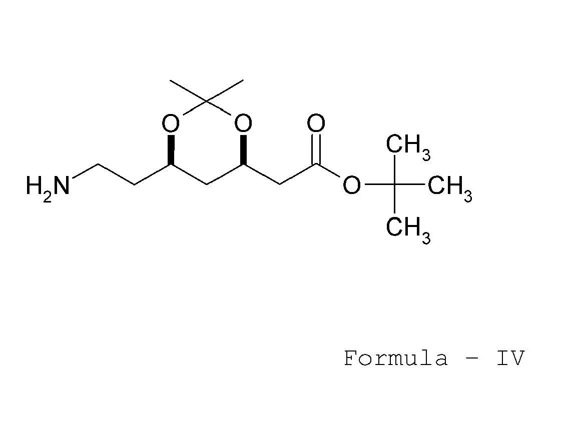

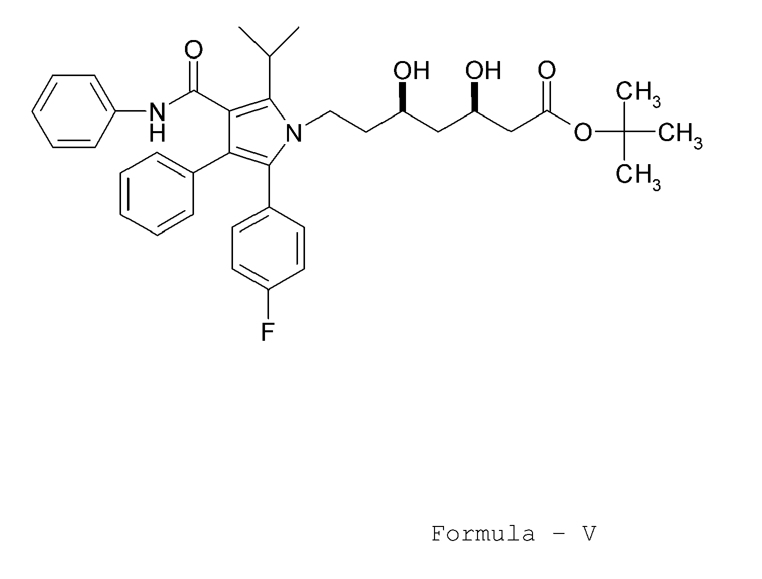

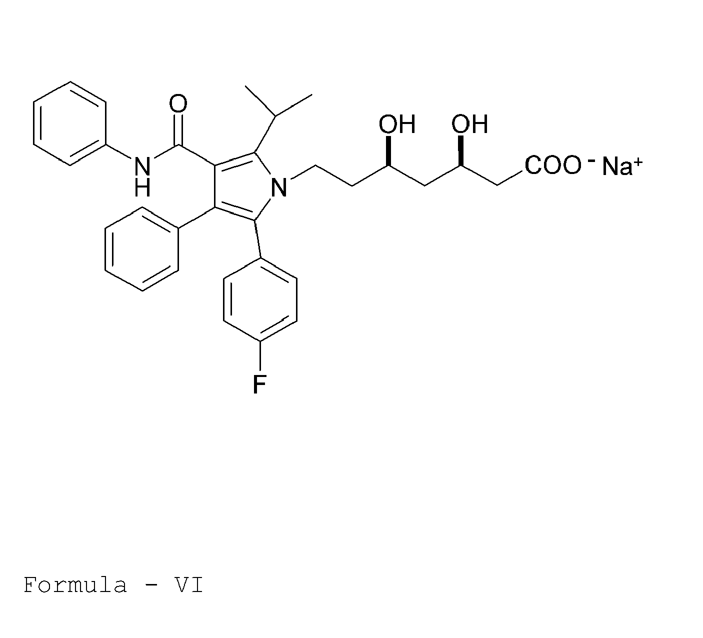

In accordance with the present invention there is provided a process for preparation of atorvastatin hemi-calcium salt of Formula I in its amorphous form comprising the following steps: 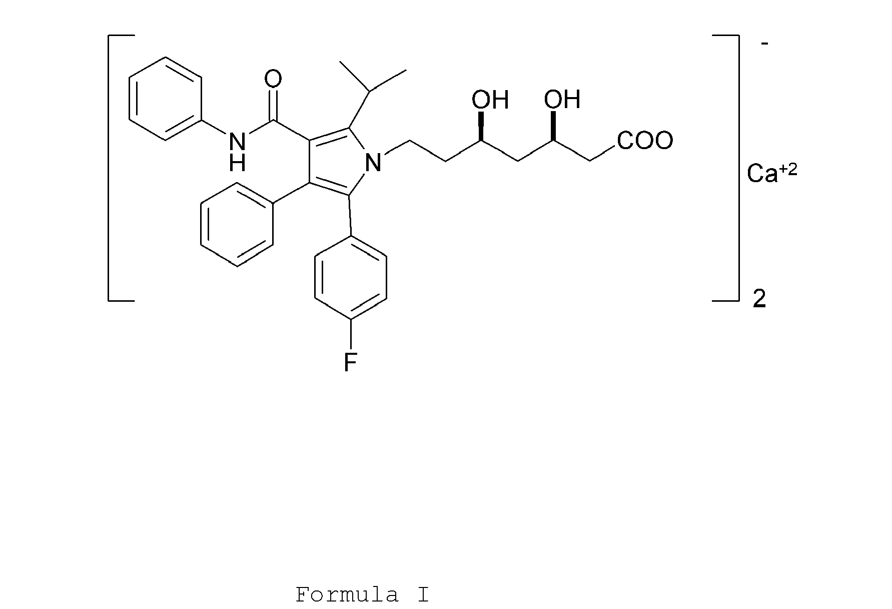
o hydrolyzing, with hydrochloric acid, a solution of the compound of Formula II in a first solvent to obtain a solution 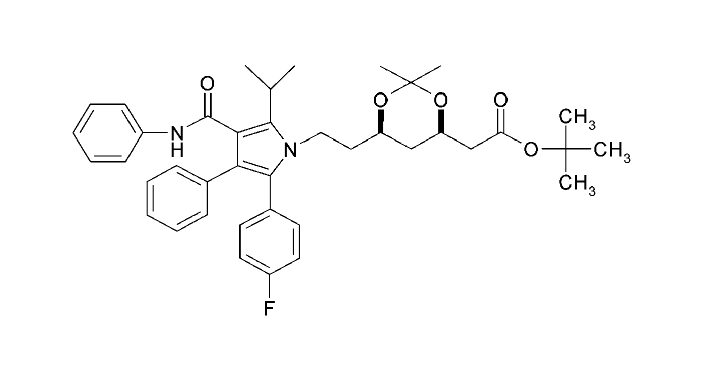 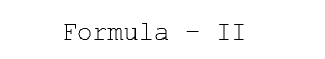 containing an intermediate diol ester of formula V ; 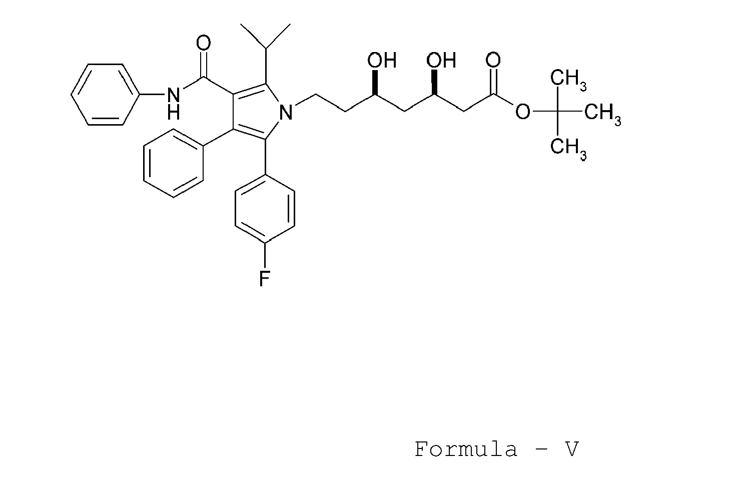
○ neutralizing the solution containing intermediate diol ester of formula V with an alkali, selected from a group of alkalis consisting of sodium hydroxide and potassium hydroxide, to obtain a neutralized solution having pH in the range of about 7.5 to about 8.00.
○ purifying the neutralized solution with the help of activated carbon treatment to obtain a first purified solution;
○ increasing the pH of the first purified solution to lie in the range of about 12 to about 12.5, by addition of sodium hydroxide and hydrolysing the solvate to obtain a solution containing atorvastatin sodium salt of formula VI; 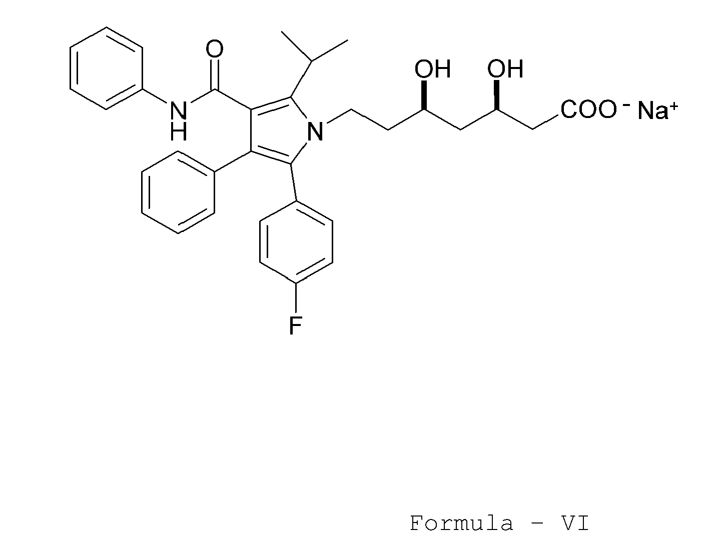
○ purifying the solution containing atorvastatin sodium salt of formula VI with activated carbon treatment to obtain a second purified solution;
○ treating the second purified solution with a calcium salt, selected from a group of calcium salts consisting of calcium acetate, calcium hydroxide and calcium chloride, in the first solvent to obtain a mixture containing atorvastatin hemicalcium salt and sodium salts ;
○ adding a second solvent to said mixture for selectively in situ dissolving atorvastatin hemicalcium salt in second solvent and removing the sodium salts by washing with water to obtain a solution containing atorvastatin hemicalcium salt in the second solvent;
○ purifying the solution of atorvastatin hemicalcium salt in the second solvent by treatment with activated carbon to obtain a third purified solution;
○ partially concentrating the third purified solution to obtain a partially concentrated third purified solution and diluting the third concentrated purified solution by addition of a third solvent to obtain a diluted solution containing atorvastatin hemicalcium salt;
○ precipitating amorphous atorvastatin hemicalcium salt by drop-wise addition of the diluted solution, in a precipitating medium, maintained at a temperature in the range of about-65°C to about 45°C, in an inert atmosphere to obtain a suspension;
○ isolating and purifying the amorphous atorvastatin hemicalcium salt by vacuum filtration followed by washing and vacuum drying at a temperature in the range of about 35°C to about 45°C.

Typically, the first solvent is at least one solvent selected from a group of solvents consisting of methanol, ethanol, propanol and tetrahydrofuran.

Typically, the second solvent is at least one solvent selected from a group of solvents consisting of ethyl acetate, methyl acetate, propyl acetate, butyl acetate, methyl butanoate, propyl butanoate, methyl propionate and ethyl propionate.

Typically, the third solvent is at least one solvent selected from a group of solvents consisting of isopropanol, ethanol, n-propanol and tetrahydrofuran.

Typically, the precipitating medium comprises
○ a hydrocarbon solvent selected from a group consisting of pentane, hexane, heptane, petroleumether, cyclohexane, methylcyclohexane and toluene,
○ an alkyl ether selected from a group of linear alkyl ethers of formula R1 -O-R2 with preferably C1 to C4 carbon atoms, and
○ at least one pharmaceutically acceptable siloxane polymer selected from the group of pharmaceutically acceptable siloxane polymers consisting of polydimethyl siloxane and simethicone.

Typically, the concentration of siloxane polymers in the precipitating medium is in the range of about 0.05% to about 0.15% by weight of the volume of the solvent medium.

In accordance with the present invention, the process as described herein below does not employ crystalline and other solid forms of atorvastatin calcium in the process for preparing amorphous atorvastatin calcium of formula -I as described hereinabove.

In accordance with the present invention there is provided a compound of Formula I characterized by its XRD pattern as an amorphous substance.
Typically the product is stored under nitrogen gas or inert gas atmosphere, under control storage temperature of 2°C to 8°C.

Also provided in accordance with this invention is a process for preparation of a compound of Formula II comprising the following method step :
- admixing compound of Formula III , compound of Formula IV, in a multi-component solvent and subjecting the resulting reaction mixture to catalytic azeotropic distillation reaction to obtain a product solution;
- cooling of the product solution followed by purification and neutralization followed by removal of the multi-component solvent to obtain a residue;
- dissolving the residue in a fourth solvent and adding a fifth solvent for precipitation followed by isolation and purification of the resulting precipitate, dissolving in a sixth solvent, subsequently removing the solvent under vacuum and at elevated temperature, cooling to ambient temperature, isolation and pulverization.

Typically, the multi- component solvent comprises at least one ether and, and at least one hydrocarbon.

Typically, the ether is at least one ether selected from the group of ethers consisting of tetrahydrofuran, and dioxane.

Typically, the hydrocarbon is at least one hydrocarbon selected from a group of hydrocarbons consisting of petroleum ether, hexane, heptane, cyclohexane, methylcyclohexane, toluene and xylene

Typically, the fourth solvent is at least one solvent selected from a group of solvents consisting of isopropanol, tethydrofuran, ethyl alcohol and water.

Typically, the fifth solvent is at least one solvent selected from a group of solvents consisting of chlorinated hydrocarbons, ethers and nitriles.

Typically, the sixth solvent is at least one solvent selected from a group of solvents consisting of dichloromethane, carbontetrachloride, tetrahydrofuran and acetonitrile.

There is also provided in accordance with this invention a compound (4R-Cis)-1,1-Dimethylethyl-6-[2[2-(4-fluorophenyl-5-(1-methylethyl)-3-phenyl-4-[(phenylamino)carbonyl]-1H-pyrrol-1-yl]ethyl]-2,2-dimethyl-1,3-dioxane-4-acetate of formula-II characterized by its XRD spectrum as an amorphous substance.

### Brief Description Of The Drawings:

The invention will be described in detail with reference to the accompanying drawings.

### In the accompanying drawings:

Figure 1 illustrates the X-Ray powder diffractogram of compound of Formula I (y-axis=counts/s x-axis= °2Theta).
Figure 2 illustrates the X-Ray powder diffractogram of compound of Formula I (y-axis=counts/s x-axis= °2Theta).
Figure 3 illustrates the X-Ray powder diffractogram of compound of Formula II (y-axis=counts/s x-axis= °2Theta).

### Detailed Description Of The Invention:

The present invention describes a process for preparation of amorphous form of atorvastatin hemi-calcium salt, compound of formula I which is beneficial for application in pharmaceutical preparation. The amorphous form is considered advantageous in pharmaceutical formulations.

The process in accordance with this invention provides atorvastatin hemi-calcium salt with homogenous amorphous character, high purity and improved stability.

The present process involves the preparation of the amorphous form while utilizing the intermediate compound of formula-II (4R-Cis)-1,1-Dimethylethyl-6-[2[2-(4-fluorophenyl-5-(1-methylethyl)-3-phenyl-4-[(phenylamino)carbonyl]-1H-pyrrol-1-yl]ethyl]-2,2-dimethyl-1,3-dioxane-4-acetate in either crystalline, amorphous or mixed forms.

In accordance with this invention, a process for preparation of amorphous form of atorvastatin hemi-calcium salt of Formula I comprises the following steps : 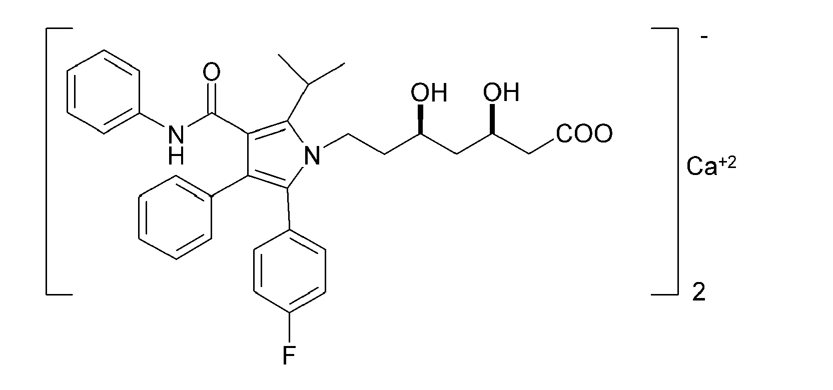 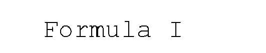 Method Step a involves hydrolyzing, with hydrochloric acid, a solution of the compound of Formula II in a first solvent to obtain a solution. 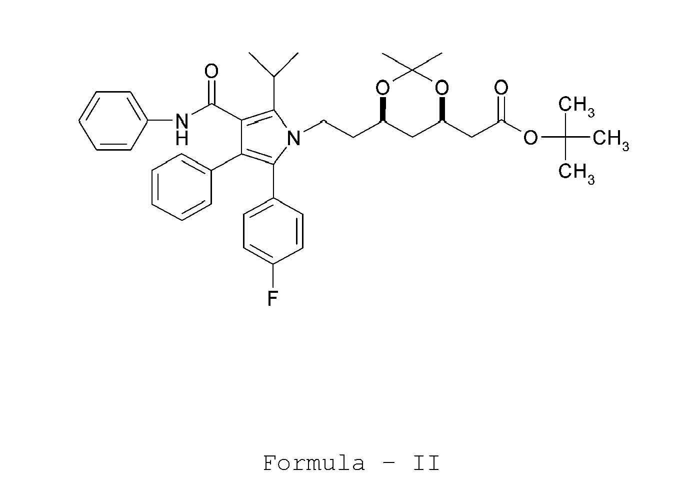 containing an intermediate diol ester of formula V

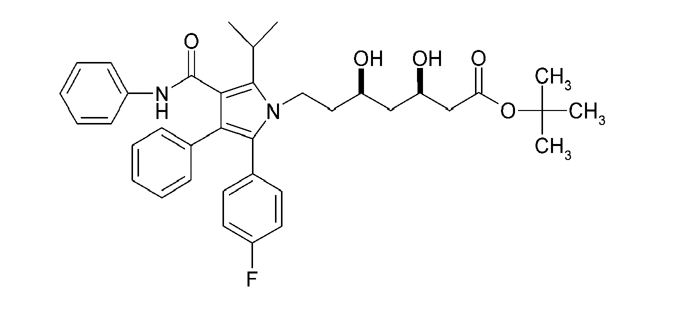

Compound of formula (II) is sequentially hydrolyzed in the presence of a first solvent, using acidic and alkaline media, to open up the protected ketal, generating the diol compound of formula V. Typically, the first solvent is at least one solvent selected from a group of solvents consisting of alcohol, ether and water. Typically, alcohols that are used as the first solvent are methanol, ethanol and propanol. Typically, ether that is used as a first solvent is tetrahydrofuran. Typically, aqueous hydrochloric acid is used for maintaining the acidic medium.

Method Step b involves neutralizing the solution containing intermediate diol ester of formula V with an alkali, selected from a group of alkalis consisting of sodium hydroxide and potassium hydroxide, to obtain a neutralized solution having pH in the range of about 7.5 to about 8.00.
Preferably, sodium hydroxide is used as an alkali.

Method step c involves purifying the neutralized solution with the help of activated carbon treatment to obtain a first purified solution;
To the neutralized solution activated carbon is added, the solution is stirred for half an hour and then it is filtered to remove the suspended particles.

Method Step d involves increasing the pH of the first purified solution to lie in the range of about 12 to about 12.5, by addition of sodium hydroxide and hydrolysing the solvate to obtain a solution containing atorvastatin sodium salt of formula VI;

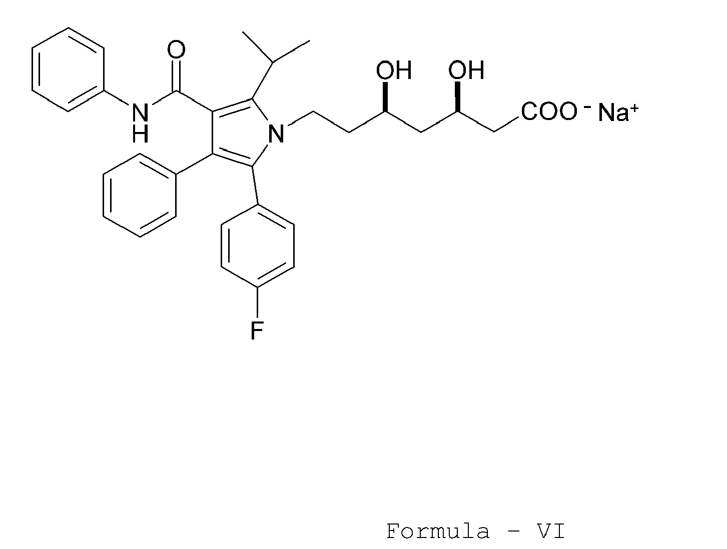

Method step e involves purifying the solution containing atorvastatin sodium salt of formula VI with activated carbon to obtain a second purified solution;

Method step f involves treating the second purified solution with a calcium salt, selected from a group of calcium salts consisting of calcium acetate, calcium hydroxide and calcium chloride, in the first solvent to obtain a mixture containing atorvastatin hemicalcium salt and sodium salts.

Method step g involves adding a second solvent to said mixture for selectively in situ dissolving atorvastatin hemicalcium salt in second solvent and removing the sodium salts by washing with water to obtain a solution containing atorvastatin hemicalcium salt in the second solvent.
Typically, the second solvent is at least one solvent selected from a group of solvents consisting of ethyl acetate, methyl acetate, propyl acetate, butyl acetate, methyl butanoate, propyl butanoate, methyl propionate and ethyl propionate.

Method step h involves purifying the solution of atorvastatin hemicalcium salt in the second solvent by treatment with activated carbon to obtain a third purified solution.
The solution of atorvastatin hemi-calcium salt in an organic solvent, which has been purified earlier by extraction and treatment with activated carbon, is dried over desiccant agents like anhydrous sodium sulphate or anhydrous magnesium sulphate and then molecular sieves.

Method step i involves partially concentrating the third purified solution to obtain a partially concentrated third purified solution and diluting the third concentrated purified solution by addition of a third solvent to obtain a diluted solution containing atorvastatin hemicalcium salt.
Typically, the third solvent is at least one solvent selected from a group of solvents consisting of alcohol and cyclic ether.
Typically when the third solvent is alcohol it is at least one alcohol selected from a group of alcohols consisting of isopropanol, ethanol, and n-propanol.
Alternatively, tetrahydrofuran is used as a third solvent.

Method step j involves precipitating amorphous atorvastatin hemicalcium salt by drop-wise addition of the diluted solution, in a precipitating medium, maintained at a temperature in the range of about-65°C to about 45°C, in an inert atmosphere to obtain a suspension.
Typically, the precipitating medium comprises a hydrocarbon solvent, an alkyl ether and a pharmaceutically acceptable siloxane polymer.
Typically, the hydrocarbon solvent is at least one solvent selected from a group of hydrocarbon solvents consisting of pentane, hexane, heptane, petroleumether, cyclohexane, methylcyclohexane and toluene.
Typically, the alkyl ether is at least one linear alkyl ether selected from a group of linear alkyl ethers of formula R1 -O-R2 with preferably C1 to C4 carbon atoms.
It has also been observed that use of certain pharmaceutically acceptable agents like polydimethyl siloxane (dimethione), simethicone (activated dimethicone), or other such silicones in low concentration such as 0.02% to 1.0% preferably 0.05% to 0.15% w/v w.r.t. the volume of precipitation solvent. Such silicone agents, seem to improve product characteristics like color and stability of the amorphous product. Stability data of amorphous atorvastatin calcium prepared as per this method was generated under accelerated stability conditions [Temperature 40°C, ±2°C, Relative humidity 75%, ±5%, period 3 months], and was compared with that of the sample prepared without addition of simethione. The fall in purity level for a sample prepared with simethicone addition was only 2.58% (Initial value 99.60%, after 3 months 97.02%), as compared to 3.38 % for the sample prepared without simethicone addition (Initial value, 99.65%, after 3 months 96.27%). Thus, rate of degradation of the product was found to be lesser by 0.8%. This is considered advantageous for the products stability characteristics and behavior. Siloxane polymers like dimethicone, simethicone etc being well established pharmaceutical agents and being practically inert, are considered acceptable for such use, especially at given low concentrations.
Typically, the pharmaceutically acceptable siloxane polymer is at least one pharmaceutically acceptable siloxane polymer selected from the group of pharmaceutically acceptable siloxanes consiting of polydimethyl siloxane and simethicone. Typically, the concentration of silicone agents in the precipitating medium is in the range of about 0.02% to about 1.0% by weight of the volume of the solvent medium.
Preferably, the concentration of silicone agents in the precipitating medium is in the range of about 0.05% to about 0.15% by weight of the volume of the solvent medium.
Typically, the precipitating medium is maintained at a temperature in the range of about -65°C to about 45°C. Preferably, the precipitating medium is maintained at a temperature in the range of about -50°C to about 30°C.
The process of amorphisation by mixing the solution of atorvastatin hemi-calcium salt with relatively lower polar solvents like hydrocarbons or ethers as described above, is carried out under inert atmosphere using nitrogen gas or other inert gas for blanketing. The mixing is carried out preferably by gradual addition of atorvastatin hemi-calcium salt solution into the vessel containing lower polar solvent under mechanized agitation.
Typically, the method step of precipitating is carried out under inert atmosphere with nitrogen gas or an inert gas.

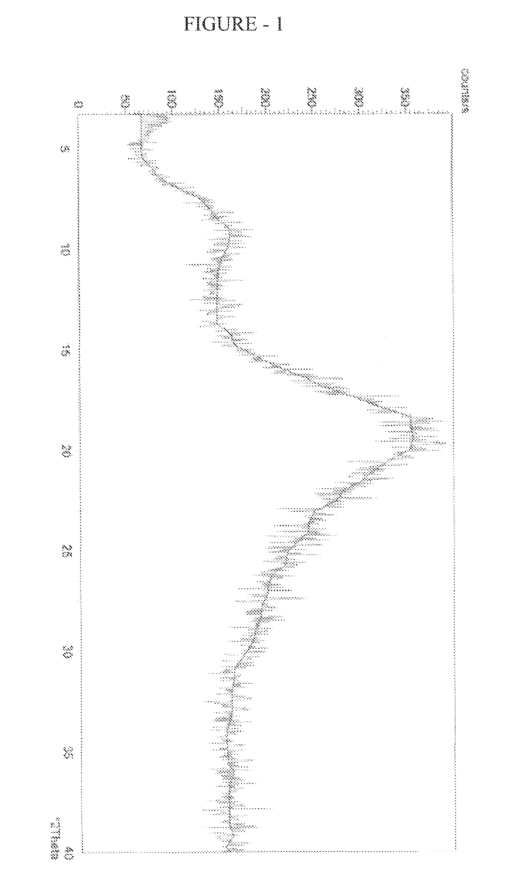

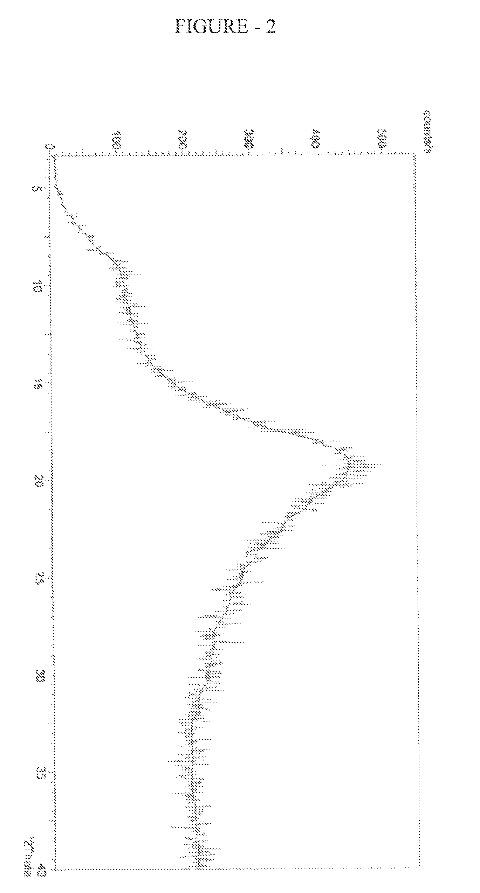

Method step k involves isolating and purifying the amorphous atorvastatin hemicalcium salt by vacuum filtration followed by washing and vacuum drying at a temperature in the range of about 35 °C to about 45°C.
The precipitated amorphous product is isolated by vacuum filtration under nitrogen gas or an inert gas atmosphere. The product cake is washed with the same low polar solvent containing the silicone agent and sucked dry to maximum extent. It is dried in vacuum at about 30 to about 55°C, preferably at about 35°C to about 45°C for several hours. The dried product is stored in sealed, low porosity containers under nitrogen or an inert gas cover at controlled temperature range of 2°C - 8°C.
The product atorvastatin hemi-calcium prepared as per above procedure is tested by powder X-ray diffraction method and the diffractogram obtained clearly depicts complete amorphous character of the product (Figures 1 and 2). The process in accordance with this invention does not employ crystalline and other solid forms of atorvastatin calcium.
The final product obtained in accordance with the procedure as hereinabove described is stored under nitrogen gas or inert gas atmosphere, under controlled storage temperature in the range of about of 2°C to about 8°C.

### Process for preparation of Compound of formula II

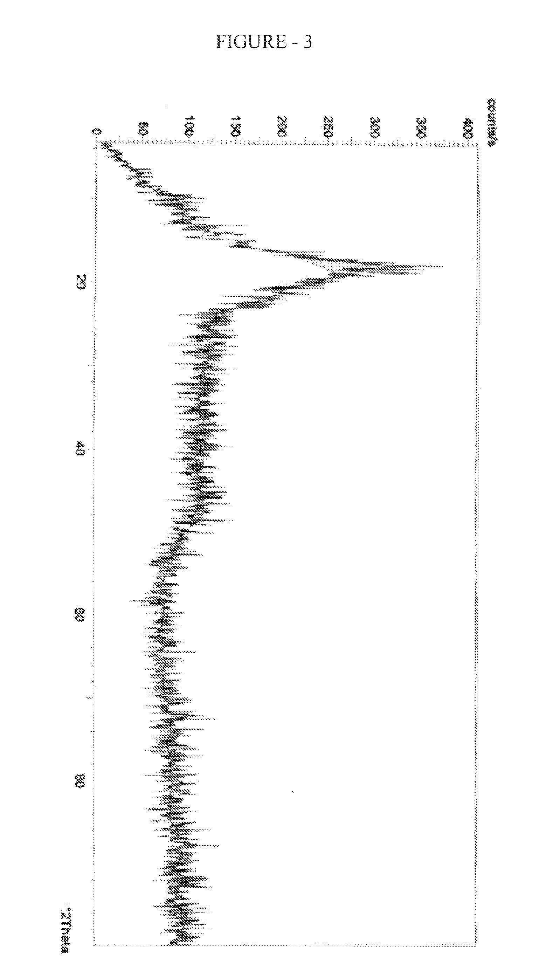

In accordance with this invention a process for preparation of a compound of Formula II comprises the following method steps:
• admixing compound of Formula III, compound of Formula IV, in a multi-component solvent and subjecting the resulting reaction mixture to catalytic azeotropic distillation reaction to obtain a product solution;
   Typically, the multi- component solvent comprises of at least one ether, and at least one hydrocarbon.
   Typically, the ether is at least one ether selected from the group of ethers consisting of tetrahydrofuran, and dioxane.
   Typically, the hydrocarbon is at least one hydrocarbon selected from a group of hydrocarbons consisting of petroleum ether, hexane, heptane, cyclohexane, methylcyclohexane, toluene and xylene.
• cooling of the product solution followed by purification and neutralization followed by removal of the multi-component solvent to obtain a residue;
• dissolving the residue in a fourth solvent and adding a fifth solvent for precipitation followed by isolation of the resulting precipitate followed by dissolution in a sixth solvent removal of the solvent under vacuum and at elevated temperature, subsequent cooling at ambient temperature, isolation and pulverization,
   Typically, the fourth solvent is at least one solvent selected from a group of solvents consisting of isopropanol, tethydrofuran, ethyl alcohol and water.
   Typically, the fifth solvent is at least one solvent selected from the group of solvents consisting of chlorinated hydrocarbons, ethers and nitriles.
   Typically, the sixth solvent is at least one solvent selected from a group of solvents consisting of dichlromethane, carbontetrachloride, tetrahydrofuran and acetonitrile.
   The compound (4R-Cis)-1,1-Dimethylethyl-6-[2[2-(4-fluorophenyl-5-(1-methylethyl)-3-phenyl-4-[(phenylamino)carbonyl]-1H-pyrrol-1-yl]ethyl]-2,2-dimethyl-1,3-dioxane-4-acetate of formula-II characterized by its XRD spectrum as an amorphous substance as shown in figure 3.

The present invention will now be illustrated by the following examples, which are not intended to limit effective scope of the claims. As a consequence, any variations of the invention described above are not to be regarded as departure from the spirit and the scope of invention as claimed. The present invention has been described in terms of its specific embodiments, and for those skilled in the art various modifications, parallels and equivalents will be apparent and are intended to be included within the scope of present invention.

### Example : 1

[R-(R*,R*)]-2(4-fluorophenyl)-β,δ-dihydroxy-5-(1-methylethyl)-3-phenyl-4-[(phenylamino)carbonyl]-1H-pyrrole-1-heptanoic acid hemi-calcium salt, (Formula I)
Preparation of atorvastatin hemi-calcium salt (formula I) solution is described as under:
Compound of formula II 15 gm (0.023 mole), was dissolved in 340 ml methanol, and 18.5 ml 2N aqueous HCl was added at room temperature. The reaction mass was stirred at room temperature for 6 to 9 hrs while the reaction was monitored by TLC.
Aqueous 10% NaOH solution was added to neutralize the above solution. To this neutralized solution sufficient active carbon was added, the solution was stirred for 30 minutes and then filtered to remove suspended materials. Filtrate was collected in a reaction vessel and then aqueous 10% NaOH soln is added to increase pH up to 12 to 12.5 . The reaction mass is stirred for several hours while monitoring the reaction by TLC. The reaction mass is filtered to remove suspended matter and concentrated under vacuum to around 85 to 100 ml and then methanol, water and methyl-tert-butyl ether were added in ratio as approximetly 1:3:6 v/v. The mass was stirred and allowed to settle, and aqueous layer was separated and collected in a vessel. The organic layer was extracted with 25 ml water and the aqueous layer was pooled up with earlier aqueous layer. Combined aqueous layer was washed with methyl-tert-butyl-ether, and then neutralized with dilute aqueous HCl to adjust pH up to 7.5 to 8.0 Then 150 ml ethyl acetate was added, the mass is stirred for 15 minutes, and 2.4 gm calcium acetate in 25 ml demineralised water. The reaction mass was stirred for 2 hrs, allowed to settle and organic and aqueous layers were separated. The aqueous layer was again extracted twice with sufficient ethyl acetate. The organic layers were combined, washed with demineralised water and collected. It was treated with active carbon, and filtered to remove suspended impurities. Filtrate was dried over molecular sieves, re-filtered and then concentrated at 40°C to 45°C in vacuum, to approximately 90 to 120 ml volume. It was used for the next stage of precipitation.

### Example : 2

[R-(R*,R*)]-2(4-fluorophenyl)-β,δ-dihydroxy-5-(1-methylethyl)-3-phenyl-4-[(phenylamino)carbonyl]-1H-pyrrole-1-heptanoic acid hemi-calcium salt, (Formula I)
Preparation of atorvastatin hemi-calcium salt (formula I) solution is described as under:
Compound of formula II 15 gm (0.023 mole), was dissolved in 280 ml methanol, and 16 ml 2.2N aqueous HCl was added slowly at room temperature. The reaction mass was stirred at room temperature for 6 to 9 hours while the reaction was monitored by TLC.
Aqueous 12% NaOH solution was added to neutralize the above solution. To this neutralized solution sufficient active carbon was added, the solution was stirred for 30 minutes and then filtered to remove suspended materials. Filtrate was collected in a reaction vessel and then aqueous 12% NaOH soln is added to increase pH up to 12 to 12.5 . The reaction mass is stirred for several hours while monitoring the reaction by TLC. The reaction mass is filtered to remove suspended matter and concentrated under vacuum to around 85 to100ml and then methanol, water and methyl-tert-butyl ether were added in ratio as approximetly 1.1:3:6.2 v/v. The mass was stirred and allowed to settle, and aqueous layer was separated and collected in a vessel. The organic layer was extracted with 25 ml water and the aqueous layer was pooled up with earlier aqueous layer. Combined aqueous layer was washed with methyl-tert-butyl-ether, and then neutralized with dilute aqueous HCl to adjust pH up to 7.5 to 8.0 Then 150 ml ethyl acetate was added, vthe mass is stirred for 15 minutes, and 2.26 gm calcium acetate in 23 ml demineralised water. The reaction mass was stirred for 2 hrs, allowed to settle and organic and aqueous layers were separated. The aqueous layer was again extracted twice with sufficient ethyl acetate. The organic layers were combined, washed with demineralised water and collected. It was treated with active carbon, and filtered to remove suspended impurities. Filtrate was dried over molecular sieve, re-filtered and then concentrated at 40°C to 45°C in vacuum, to approximately 90 to 120 ml volume. In this solution 23 ml tetrahydrofuran was added and was used for the next stage of precipitation.

### Example : 3

[R-(R*,R*)]-2(4-fluorophenyl)-β,δ-dihydroxy-5-(1-methylethyl)-3-phenyl-4-[(phenylamino)carbonyl]-1H-pyrrole-1-heptanoic acid hemi-calcium salt, (Formula I)
Preparation of atorvastatin hemi-calcium salt (formula I) solution is described as under:
15 gms of compound of formula-II (4R-Cis)-1,1-Dimethylethyl-6-[2[2-(4-fluorophenyl-5-(1-methylethyl)-3-phenyl-4-[(phenylamino)carbonyl]-1H-pyrrol-1-yl]ethyl]-2,2-dimethyl-1,3-dioxane-4-acetate is dissolved in 325 ml of methanol and 18 ml 2N aqueous hydrochloric acid is added. The reaction mass was stirred for 6-9 hrs at room temperature (30 - 35°C). 39.5 ml 10% methanolic sodium hydroxide is then added gradually to the reaction mixture at room temperature. The reaction mass is then stirred further while reaction completion is monitored by TLC. The pH of the reaction mixture is then adjusted to 7.5 to 8 by 2N methanolic hydrochloric acid, active carbon is added in the reaction mass , stirred for 30 minutes and filtered to remove suspended matter. The filtrate is then heated to 45 - 50°C , solution of 1.56 gms calcium chloride in 16 ml methanol is added and stirred the solution for 2 hrs. The reaction mass is then cooled to room temperature, active carbon is added, stirred for 1 hr. and filtered through the 0.5 µ filter. Approximately 80% to 90% methanol is then distilled off, under vacuum. 270 ml ethyl acetate is added in the flask and the mass is stirred for 1 hr. The reaction mass is filtered to remove any suspended matter, washed with demineralised water and layers are separated. Ethyl acetate layer is dried over molecular sieve, filtered, repurified by carbon treatment, filtered and concentrated to 135 ml under vacuum and stored for next step.

### Example : 4

[R-(R*,R*)]-2(4-fluorophenyl)-β,δ-dihydroxy-5-(1-methylethyl)-3-phenyl-4-[(phenylamino)carbonyl]-1H-pyrrole-1-heptanoic acid hemi-calcium salt, (Formula II)
Preparation of atorvastatin hemi-calcium salt (formula I) solution is described as under:
13.9 gms of compound of formula-II (4R-Cis)-1,1-Dimethylethyl-6-[2[2-(4-fluorophenyl-5-(1-methylethyl)-3-phenyl-4-[(phenylamino)carbonyl]-1H-pyrrol-1-yl]ethyl]-2,2-dimethyl-1,3-dioxane-4-acetate is dissolved in 340 ml of methanol and 17.4 ml 2N methanolic hydrochloric acid is added. The reaction mass was stirred for 6-9 hrs at room temperature (30 - 35°C). 38.0ml 10% methanolic sodium hydroxide is then added gradually to the reaction mixture at room temperature. The reaction mass is then stirred further while reaction completion is monitored by TLC. The pH of the reaction mixture is then adjusted to 7.5 to 8 by 2N methanolic hydrochloric acid, active carbon is added in the reaction mass , stirred for 30 minutes and filtered to remove suspended matter. The filtrate is then heated to 45 - 50°C, solution of 1.45 gms calcium chloride in 14 ml methanol is added and stirred the solution for 2 hrs. The reaction mass is then cooled to room temperature, active carbon is added, stirred for 1 hr. and filtered through the 0.5 µ filter. Approximately 80% to 85% methanol is then distilled off, under vacuum. 285 ml methyl acetate is added in the flask and the mass is stirred for 1 hr. The reaction mass is filtered to remove any suspended matter, washed with demineralised water and layers are separated. Methyl acetate layer is dried over molecular sieve, filtered , re-purified by carbon treatment, filtered and concentrated to 122 ml under vacuum, added with 25 ml tetrahydrofuran and stored for next step.

### Example : 5

[R-(R*,R*)]-2(4-fluorophenyl)-β,δ-dihydroxy-5-(1-methylethyl)-3-phenyl-4-[(phenylamino)carbonyl]-1H-pyrrole-1-heptanoic acid hemi-calcium salt, (Formula I)
Preparation of atorvastatin hemi-calcium salt (formula I) solution is described as under:
14.1 gms of compound of formula-II (4R-Cis)-1,1-Dimethylethyl-6-[2[2-(4-fluorophenyl-5-(1-methylethyl)-3-phenyl-4-[(phenylamino)carbonyl]-1H-pyrrol-1-yl]ethyl]-2,2-dimethyl-1,3-dioxane-4-acetate is dissolved in 335 ml of methanol and 15.25ml 2.1N aqueous hydrochloric acid is added. The reaction mass was stirred for 6-9 hrs at room temperature (30 - 35°C). 38.5 ml 10% methanolic sodium hydroxide is then added gradually to the reaction mixture at room temperature.

The reaction mass is then stirred further while reaction completion is monitored by TLC. The pH of the reaction mixture is then adjusted to 7.5 to 8 by 2N methanolic hydrochloric acid, active carbon is added in the reaction mass , stirred for 30 minutes and filtered to remove suspended matter. The filtrate is then heated to 48 - 50°C, solution of 1.56 gms calcium chloride in 16 ml methanol is added and stirred the solution for 2 hrs. The reaction mass is then cooled to room temperature, active carbon is added, stirred for 1 hr. and filtered through the 0.5 µ filter. Approximately 80% to 90% methanol is then distilled off, under vacuum. 290 ml ethyl acetate is added in the flask and the mass is stirred for 1 hr. The reaction mass is filtered to remove any suspended matter, washed with demineralised water and layers are separated. ethyl acetate layer is dried over molecular sieve, filtered , repurified by carbon treatment, filtered and concentrated to 115 ml under vacuum ,added 20 ml tetrahydrofuran and stored for next step.

### Example : 6

[R-(R*,R*)]-2(4-fluorophenyl)-β,δ-dihydroxy-5-(1-methylethyl)-3-phenyl-4-[(phenylamino)carbonyl]-1H-pyrrole-1-heptanoic acid hemi-calcium salt, (Formula I)
Preparation of atorvastatin hemi-calcium salt (formula I) solution is described as under:
14.25 gms of compound of formula-II (4R-Cis)-1,1-Dimethylethyl-6-[2[2-(4-fluorophenyl-5-(1-methylethyl)-3-phenyl-4-[(phenylamino)carbonyl]-1H-pyrrol-1-yl]ethyl]-2,2-dimethyl-1,3-dioxane-4-acetate is dissolved in 340 ml of methanol and 16.7ml 2.1N aqueous hydrochloric acid is added.

The reaction mass was stirred for 6-9 hrs at room temperature (30 - 35°C). 39.5 ml 10% methanolic sodium hydroxide is then added gradually to the reaction mixture at room temperature. The reaction mass is then stirred further while reaction completion is monitored by TLC. The pH of the reaction mixture is then adjusted to 7.5 to 8 by 2N methanolic hydrochloric acid, active carbon is added in the reaction mass , stirred for 30 minutes and filtered to remove suspended matter. The filtrate is then heated to 45 - 50°C , solution of 1.52 gms calcium chloride in 16 ml methanol is added and stirred the solution for 2 hrs. The reaction mass is then cooled to room temperature, active carbon is added, stirred for 1 hr. and filtered through the 0.5 µ filter. Approximately 80% to 90% methanol is then distilled off, under vacuum. 320 ml ethyl acetate is added in the flask and the mass is stirred for 1 hr. The reaction mass is filtered to remove any suspended matter, washed with demineralised waterand layers are separated. Ethyl acetate layer is dried over molecular sieve, filtered , repurified by carbon treatment, filtered and concentrated to 135 ml under vacuum and stored for next step.

### Preparation of amorphous atorvastatin hemi-calcium salt (I)

### Example : 7

Solution of atorvastatin hemi-calcium salt prepared as per example 1 is then drop-wise added to methyl cyclohexane (600ml) under mechanical stirring at ambient temperature (28°C - 30°C) under inert atmosphere with nitrogen gas. Addition is completed in about one hour. During the addition of atorvastatin hemi-calcium solution to methyl cyclohexane, precipitation of amorphous atorvastatin calcium salt begins. After completion of solution addition, the suspension is further stirred for 1 hour at room temperature.
The suspended product obtained was filtered and washed with 75ml methylcyclohexane. White product was dried in vacuum oven at 40°C-45°C for 12 hrs.
The product obtained was characterized as amorphous atorvastatin calcium based on its X-ray powder diffraction pattern.
Yield 11.5 gms, HPLC purity- 99.64 %

### Example -8

Solution of atorvastatin hemi-calcium salt prepared as per example 1 is then drop-wise added to methyl cyclohexane (600ml) containing 0.075% (w/v) of polydimethylsiloxane under mechanical stirring at room temperature and inert atmosphere with nitrogen gas. Addition continued for one hour. During addition of atorvastatin hemi-calcium solution to methyl cyclohexane the precipitation of amorphous atorvastatin calcium salt begins. After completion of solution addition, the suspension is stirred for1 hour at room temperature.The product obtained was filtered, and wash by 75ml methylcyclohexane containing 0.0750 (w/v) polydimethylsiloxane. The fine white product was dried in vacuum oven at 40°-45°C for 12 hrs.
The powdered material obtained was characterized as amorphous Atorvastatin calcium based on its X-ray powder diffraction pattern.
Yield 11.7 gms, HPLC purity- 99.65 %

### Example -9

Solution of atorvastatin hemi-calcium salt prepared as per example 1 is then added slowly to methyl cyclohexane (600ml) containing 0.09% (w/v) simethicone under mechanical stirring at -60 °C under inert atmosphere with nitrogen gas. Addition takes around one hour. During addition of atorvastatin hemi-calcium solution to methyl cyclohexane the precipitation of amorphous atorvastatin calcium salt begins. After completion of solution addition, the suspension is further stirred at (-)50°C to (-)60°C and for 30 minutes at room temperature.
The product obtained was filtered and washed with 75ml methylcyclohexane containing 0.09% (w/v) simethicone. The fine white product was dried in vacuum oven at 40°-45°C for 12 hrs. The powdered material obtained was characterized as amorphous Atorvastatin calcium based on its X-ray powder diffraction pattern as shown in Figure 1.
Yield 11.8gm, HPLC purity- 99.65 %

### Example -10

Solution of atorvastatin hemi-calcium salt prepared as per example 1 is then drop-wise added to methyl cyclohexane 540ml and 60ml dry toluene containing 0.08% (w/v) polydimethylsiloxane under mechanical stirring at -60°C under inert atmosphere with nitrogen gas. Addition was completed in 40 minutes. The suspension of the amorphous product precipitated thus is further stirred for about one hour at (-)50°C to (-)60°C.
The product obtained was filtered rapidly and washed by chilled 75ml methylcyclohexane containing 0.08% (w/v) of polydimethylsiloxane. The fine white product was dried in vacuum oven at 40° - 45°C for 12 hrs.
The powdered material obtained was characterized as amorphous Atorvastatin calcium based on its X-ray powder diffraction pattern as shown in Fig 1
Yield 11.6gms, HPLC purity- 99.66 %

### Example -11

Solution of atorvastatin hemi-calcium salt prepared as per example 1 is then dropwise added to a mixture of methyl cyclohexane 540ml and 60ml dry toluene containing 0.10% (w/v) simethicone under mechanical stirring at (-)40°C under inert atmosphere with nitrogen gas. Addition takes around 1 hr. During addition of Atorvastatin hemi-calcium solution to methyl cyclohexane the precipitation of amorphous Atorvastatin calcium salt begins. After completion of solution addition, the suspension was further stirred for 1 hour at (-)30°C to (-)40°C.
The product obtained was rapidly filtered and washed by chilled 75 ml methylcyclohexane containing 0.10% (w/v) simethicone. The fine white product was dried in vacuum oven at 40° - 45°C for 12 hrs
The powdered material obtained was characterized as amorphous Atorvastatin calcium based on its X-ray powder diffraction pattern.
Yield 11.7gm, HPLC purity- 99.61 %

### Example -12

Solution of atorvastatin hemi-calcium salt prepared as per example 1 is then drop-wise added to heptane 540ml and 60ml dry toluene containing 0.05% (w/v) polydimethylsiloxane under continued mechanical stirring at (-)40 °C under inert atmosphere with nitrogen gas. During addition of atorvastatin hemi-calcium, the precipitation of amorphous atorvastatin calcium salt begins. After completion of solution addition, the suspension is further stirred for 1 hour. The solvent was removed under stirring by vacuum distillation at ambient temperature under continued mechanical agitation.
The fine white product obtained was dried in vacuum oven at 40° - 45°C for 12 to 14 hrs.
The powdered material obtained was characterized as amorphous Atorvastatin calcium based on its X-ray powder diffraction pattern.
Yield 11.9gm, HPLC purity- 99.32 %

### Example -13

Solution of atorvastatin hemi-calcium salt prepared as example 2 is then drop-wise added to 700 ml dry cyclohexane, under very efficient mechanical stirring at 0 to 5°C in inert atmosphere with nitrogen gas. During addition of atorvastatin hemi-calcium solution to cyclohexane, the precipitation of amorphous Atorvastatin calcium salt starts developing rapidly. After completion of solution addition, the suspension is further stirred for 1 hour.
The product obtained was filtered, and washed with 75ml cyclohexane. The fine white product was dried in vacuum oven at 40 - 45°C for 10-12 hrs.
The powdered material obtained was characterized as amorphous atorvastatin calcium based on its X-ray powder diffraction pattern.
Yield 11.1gm, HPLC purity- 99.57 %.

### Example -14

Solution of atorvastatin hemi-calcium salt prepared as per example 2 is then dropwise added to 700ml dry petroleum ether (60 - 80°C), with continued mechanical stirring at room temperature under inert atmosphere with nitrogen gas. Addition takes about 1 hr. During addition of atorvastatin hemi-calcium solution to petroleum ether, the precipitations of amorphous atorvastatin calcium salt begins. After completion of addition of solution, the suspension further stirred for 1 hour at room temperature.
The product obtained was filtered and washed with 75ml petroleum ether. The fine white product was dried in vacuum oven at 40°-45°C for 10 - 12 hours.The powdered material obtained was characterized as amorphous Atorvastatin calcium based on its X-ray powder diffraction pattern as shown in Fig 1
Yield 13.8gms, HPLC purity- 99.51%

### Example :15

Solution of atorvastatin hemi-calcium salt prepared as example 4 is then drop-wise added to 825 ml dry cyclohexane containing 0.08% v/v polydimethylsiloxane, under very efficient mechanical stirring at 0 to 5°C in inert atmosphere with nitrogen gas. During addition of atorvastatin hemi-calcium solution to cyclohexane, the precipitation of amorphous Atorvastatin calcium salt starts developing rapidly. After completion of solution addition, the suspension is further stirred for 1 hour.

The product obtained was filtered, and washed with 75ml cyclohexane containing 0.08% polydimethylsiloxane. The fine white product was dried in vacuum oven at 40 - 45°C for 10-12 hrs.
The powdered material obtained was characterized as amorphous atorvastatin calcium based on its X-ray powder diffraction pattern as shown in Fig 1.
Yield 10.9gm, HPLC purity- 99.40 %.

### Example-16

Solution of atorvastatin hemi-calcium salt prepared as example 5 is then drop-wise added to 670 ml heptane containing 0.06% polydimethylsiloxane, under very efficient mechanical stirring at 0 to 5°C in inert atmosphere with nitrogen gas. During addition of atorvastatin hemi-calcium solution to heptane, the precipitation of amorphous Atorvastatin calcium salt starts developing rapidly. After completion of solution addition, the suspension is further stirred for 1 hour.
The product obtained was filtered, and washed with 75ml heptane containing 0.06% polydimethylsiloxane. The fine white product was dried in vacuum oven at 40 - 45°C for 10-12 hrs. The powdered material obtained was characterized as amorphous atorvastatin calcium based on its X-ray powder diffraction pattern as shown in Fig 2.
Yield 10.64gm, HPLC purity- 99.24 %.

### Example 17

Procedure for preparation of the pyrrole derivative (4R-Cis)-1,1-Dimethylethyl-6-[2[2-(4-fluorophenyl-5-(1-methylethyl)-3-phenyl-4-[(phenylamino)carbonyl]-1H-pyrrol-1-yl]ethyl]-2,2-dimethyl-1,3-dioxane-4-acetate (compound of formula-II) In a reaction flask was added tetrahydrofuran 180ml, toluene 235 ml and n-heptane 775 ml. To this mixture of solvents is added ± (4-fluoro-alpha-(2-methyl-1-oxopropyl)-gama-oxo-N-beta-diphenyl benzenebutaneamine of formula III(148 gms, 0.36 mole), (4R-Cis)-1,1-dimethylethyl-6-(2-aminoethyl)-2,2-dimethyl-1,3-dioxane-4-acetate (112 gms, 0.41 mole) of formula IV, pivalic acid 22 gms, and was heated to reflux for 40 hrs with azeotropic distillation arrangement to continuously remove the water produced during the reaction. The reaction mass was then cooled to ambient temperature and neutralised with saturated solution of sodium bicarbonate till nearly neutral pH . The organic layer is isolated and is purified with activated carbon. The solvent is distilled off under vacuum, and resultant residue was dissolved in 400 ml isopropyl alcohol and cooled to room temperature. To the above solution demineralised water was slowly added to precipitate the product. The precipitated product was then cooled to 20°C, stirred and filtered. It is washed several times with aqueous isopropanol. The product was dried at 40 - 45°C in a vacuum oven to give 165 gms of the title compound, which is next purified from isopropanol and water.
The product exhibits melting point 75-79 ° C and its XRD spectrum as shown in Fig 3. is in concordance with its amorphous character.

### Example 18

Procedure for preparation of the amorphous Pyrrole derivative (4R-Cis)-1,1-Dimethylethyl-6-[2[2-(4-fluorophenyl-5-(1-methylethyl)-3-phenyl-4-[(phenylamino)carbonyl]-1H-pyrrol-1-yl]ethyl]-2,2-dimethyl-1,3-dioxane-4-acetate (compound of formula-II)
In a reaction flask was added tetrahydrofuran 18 ml. toluene 24 ml, and
heptane 78 ml. To this mixture of solvents is added +/- (4-fluoro-alpha-(2-methyl-1-oxopropyl)-gama-oxo-N-beta-diphenyl benzenebutaneamine) of formula III (14.8gms,0.036 mole), (4R-Cis)-1,1-dimethylethyl-6-(2-aminoethyl)-2,2-dimethyl-1,3-dioxane-4-acetate (11.2gms, 0.041 mole) of formula IV, pivalic acid 2.2 gms, and was heated to reflux for 40 to 50 hours with azeotropic distillation arrangement to continuously remove water formed in the reaction. The reaction mass was then cooled to ambient temp and neurtralised with saturated solution of sodium bicarbonate till nearly neutral pH . The organic layer is isolated and is treated with activated carbon and filtered. The solvent is distilled off under vacuum and the resultant residue was treated with aqueous isopropyl alcohol 50 ml. After cooling to room temp, more demineralised water is added if required to precipitate the product. The product mass is next cooled to 20°C ,filtered and washed with aqueous isopropanol. It is next washed with and dissolved in sufficient quantity of dichloromethane. The dichloromethane solution is next treated with activated carbon, filtered and dried over anhydrous sodium sulphate. The solutions transferred to a round bottom flask and is slowly warmed using an oil bath. After removal of the solvent, high vacuum (1-2 mm of Hg) is applied to the flask at 100 to 110 °C for 30 minutes. The product mass is cooled to ambient temperature, vacuum is discontinued and product is removed from the flask. It is pulverized and stored in well closed containers. The product exhibits melting point 76-80 ° C and its XRD spectrum as shown in Fig 3. is in concordance with its amorphous character.

### Example 19

Procedure for preparation of the amorphous Pyrrole derivative (4R-Cis)-1,1-Dimethylethyl-6-[2[2-(4-fluorophenyl-5-(1-methylethyl)-3-phenyl-4-[(phenylamino)carbonyl]-1H-pyrrol-1-yl]ethyl]-2,2-dimethyl-1,3-dioxane-4-acetate (compound of formula-II)
The experimental procedure followed is as per Example 18, except that the quantities of tetrahydrofuran and toluene are 22ml and 30 ml respectively, while the third solvent is hexane with quantity as 90ml.

### Example 20

Procedure for preparation of the amorphous Pyrrole derivative (4R-Cis)-1,1-Dimethylethyl-6-[2[2-(4-fluorophenyl-5-(1-methylethyl)-3-phenyl-4-[(phenylamino)carbonyl]-1H-pyrrol-1-yl]ethyl]-2,2-dimethyl-1,3-dioxane-4-acetate (compound of formula-II)
The experimental procedure followed is as per Example 18, except that the filtered product mass is washed with and dissolved in sufficient quantity of tetrahydrofuran. Further,the product mass, after removal of the solvent under vacuum is heated at 100° to 115°C for a period of 45 minutes, under high vacuum(1-2 mm of Hg).

### Example 21

Procedure for preparation of the amorphous Pyrrole derivative (4R-Cis)-1,1-Dimethylethyl-6-[2[2-(4-fluorophenyl-5-(1-methylethyl)-3-phenyl-4-[(phenylamino)carbonyl]-1H-pyrrol-1-yl]ethyl]-2,2-dimethyl-1,3-dioxane-4-acetate (compound of formula-II)
The experimrntal procedure followed is as per Example 19 except that the filtered product mass is washed with and dissolved in sufficient quantity of acetonitrile, followed by desolventisation in vacuum at 100° to 105°C for a period of 45 minutes
The product obtained is homogenously amorphous and shows consistently good purity level and stability characteristics. The said process can be scaled up easily to produce the material commercially. Furthermore, this process can be scaled-up for industrial manufacturing.

## Claims

1. A process for preparation of amorphous form of atorvastatin hemi-calcium salt of Formula I comprising the following steps : 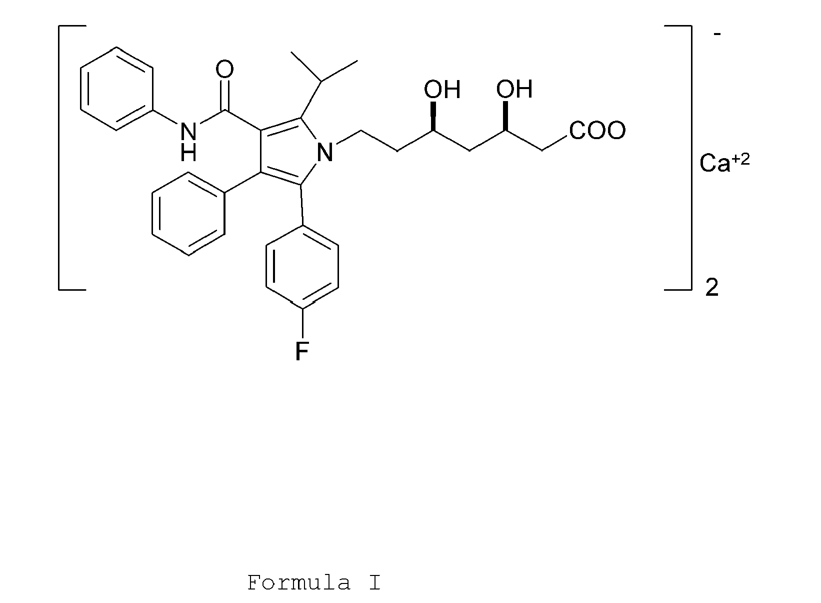
a) hydrolyzing, with hydrochloric acid, a solution of the compound of Formula II in a first solvent to obtain a solution 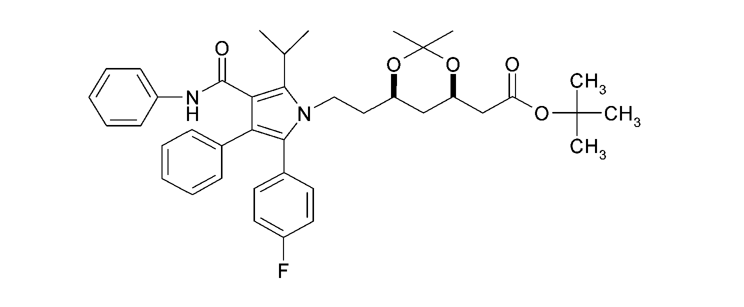 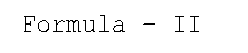 containing an intermediate diol ester of formula V ; 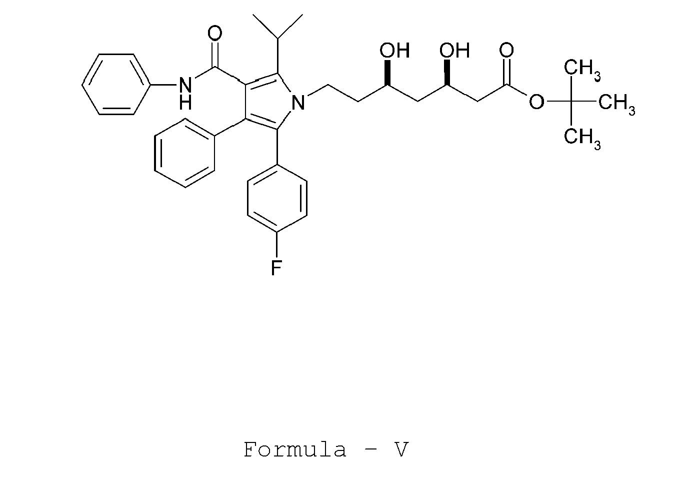
b) neutralizing the solution containing intermediate diol ester of formula V with an alkali, selected from a group of alkalis consisting of sodium hydroxide and potassium hydroxide, to obtain a neutralized solution having pH in the range of substantially 7.5 to substantially 8.00.
c) purifying the neutralized solution with the help of activated carbon treatment to obtain a first purified solution;
d) increasing the pH of the first purified solution to lie in the range of substantially 12 to substantially 12.5, by addition of sodium hydroxide and hydrolysing the solvate to obtain a solution containing atorvastatin sodium salt of formula VI; 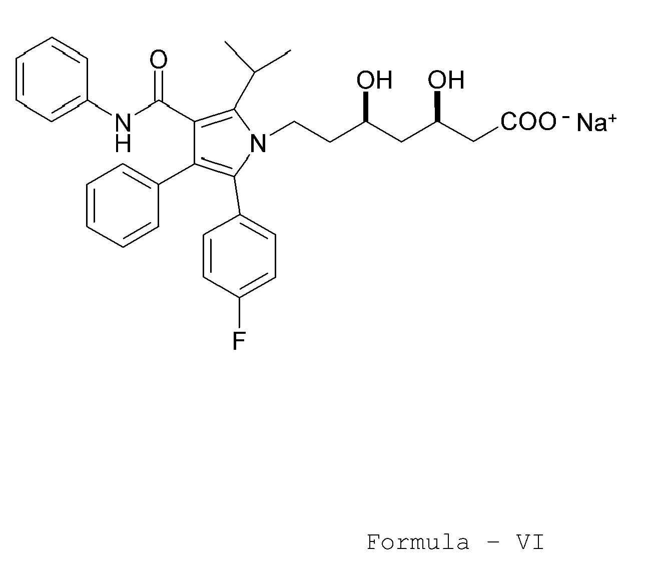
e) purifying the solution containing atorvastatin sodium salt of formula VI with activated carbon treatment to obtain a second purified solution;
f) treating the second purified solution with a solution of a calcium salt, selected from calcium acetate, calcium hydroxide and calcium chloride, in the first solvent to obtain a mixture containing atorvastatin hemicalcium salt and atorvastatin sodium salt;
g) adding a second solvent to said mixture for selectively in situ dissolving atorvastatin hemicalcium salt in second solvent and removing the sodium salts by washing with water to obtain a solution containing atorvastatin hemicalcium salt in the second solvent;
h) purifying the solution of atorvastatin hemicalcium salt in the second solvent by treatment with activated carbon to obtain a third purified solution;
i) partially concentrating the third purified solution to obtain a partially concentrated third purified solution and diluting the third concentrated purified solution by addition of a third solvent to obtain a diluted solution containing atorvastatin hemicalcium salt;
j) precipitating amorphous atorvastatin hemicalcium salt by drop-wise addition of the diluted solution, in a precipitating medium, maintained at a temperature in the range of substantially -65°C to substantially 45°C, in an inert atmosphere to obtain a suspension;
k) isolating and purifying the amorphous atorvastatin hemicalcium salt by vacuum filtration followed by washing and vacuum drying at a temperature in the range of about 35°C to about 45°C.

2. A process according to claim 1 wherein the first solvent is at least one solvent selected from methanol, ethanol, propanol and tetrahydrofuran.

3. A process according to either claim 1 or 2 wherein the second solvent is at least one solvent selected from ethyl acetate, methyl acetate, propyl acetate, butyl acetate, methyl butanoate, propyl butanoate, methyl propionate and ethyl propionate.

4. A process according to any preceding claim wherein the third solvent is at least one solvent selected from isopropanol, ethanol, n-propanol and tetrahydrofuran.

5. A process according to any preceding claim wherein, the precipitating medium comprises
i a hydrocarbon solvent selected from a group consisting of pentane, hexane, heptane, petroleumether, cyclohexane, methylcyclohexane and toluene,
ii an alkyl ether selected from linear alkyl ethers of formula R1 -O-R2 with preferably C1 to C4 carbon atoms, and
iii at least one pharmaceutically acceptable siloxane polymer selected from pharmaceutically acceptable siloxane polymers consisting of polydimethyl siloxane and simethicone.

6. A process according to claim 7, wherein the concentration of siloxane polymers in the precipitating medium is in the range of substantially 0.05% to substantially 0.15% by weight of the volume of the solvent medium.

7. A compound of Formula I **characterized by** its XRD pattern as an amorphous substance.
